# EUROPÄISCHE PATENTSCHRIFT

(11) **EP 3 413 317 B1**
(45) Veröffentlichungstag und Bekanntmachung des Hinweises auf die Patenterteilung: **17.06.2020**
(21) Anmeldenummer: 18153903.2
(22) Anmeldetag: 29.01.2018
(51) Int. Cl.: A61B 6/03

(54) **BEREITSTELLEN EINES MEDIZINISCHEN BILDES**
PROVISION OF A MEDICAL IMAGE
FOURNITURE D'UNE IMAGE MÉDICALE

(43) Veröffentlichungstag der Anmeldung: 12.12.2018
(73) Patentinhaber: Siemens Healthcare GmbH, 91052 Erlangen (DE)
(72) Erfinder: Hofmann, Christian, 91052 Erlangen (DE); Ritter, André, 91054 Buckenhof (DE)

(56) Entgegenhaltungen:
- DE-A1-102016 215 105
- US-A1- 2003 011 368
- US-A1- 2006 253 261
- US-A1- 2008 103 386
- US-A1- 2009 010 513
- US-A1- 2013 033 262
- US-A1- 2015 187 073

## Beschreibung

Die Erfindung betrifft ein Verfahren für ein Bereitstellen eines medizinischen Bildes eines Patienten, eine Anlage für ein Bereitstellen eines medizinischen Bildes eines Patienten und ein Computerprogrammprodukt.

In der medizinischen Bildgebung ist eine bildgebende Messsequenz, welche typischerweise in einer medizinischen Bildgebungsmodalität durchführbar ist, vorzugsweise derart optimiert, dass in erster Linie ein physiologischer Zustand eines Patienten in einem medizinischen Bild sichtbar sein kann. Aufgrund unterschiedlicher technischer Herausforderungen ist es üblicherweise nicht oder nur unter hohem Aufwand möglich, mehrere, insbesondere unterschiedliche, physiologische Zustände des Patienten in dem medizinischen Bild sichtbar zu machen, insbesondere wenn das medizinische Bild einen vergleichsweise großen Messbereich aufweist oder mehrere medizinische Bilder über den Messbereich aufgeteilt sind, welche jeweils die physiologischen Zustände aufweisen sollen.

In der US 2009/0 010 513 A1 sowie der US 2008/0 103 386 A1 sind verschiedene Verfahren für das Erfassen von angiographischen Zuständen mittels eines Magnetresonanztomographen offenbart.

Ein Problem kann daraus entstehen, dass erste physiologische Zustände und zweite physiologische Zustände sich in der jeweiligen Dynamik unterscheiden. Insbesondere aufgrund der unterschiedlichen Dynamik kann die bildgebende Messsequenz typischerweise entweder im Hinblick auf das Erfassen der ersten physiologischen Zustände oder auf das Erfassen der zweiten physiologischen Zustände optimiert werden. Ferner ist üblicherweise ein maximales Gesichtsfeld der medizinischen Bildgebungsmodalität kleiner als der für das gleichzeitige Erfassen der ersten physiologischen Zustände und der zweiten physiologischen Zustände notwendige Messbereich der bildgebenden Messsequenz. Sprich die ersten physiologischen Zustände und die zweiten physiologischen Zustände aufweisende medizinische Messdaten können typischerweise nacheinander, insbesondere nicht gleichzeitig, erfasst werden.

Der Erfindung liegt die Aufgabe zu Grunde, ein Verfahren für ein Bereitstellen eines medizinischen Bildes eines Patienten, eine Anlage für ein Bereitstellen eines medizinischen Bildes eines Patienten und ein Computerprogrammprodukt anzugeben, wobei das medizinische Bild unterschiedliche physiologische Zustände oder Zustandskombinationen aufweisen kann.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Bereitstellen eines medizinischen Bildes eines Patienten weist folgende Schritte auf:
- Erfassen von medizinischen Messdaten des Patienten mittels eines Computertomographen,
   -- wobei die medizinischen Messdaten eine Menge an mehreren abgetasteten Zustandskombinationen aufweisen,
   -- wobei ein erster Zustandsraum erste physiologische Zustände des Patienten aufweist, wobei ein zweiter Zustandsraum zweite physiologische Zustände des Patienten aufweist und wobei der erste Zustandsraum und der zweite Zustandsraum zusammen einen dritten Zustandsraum aufspannen,
   -- wobei jede der mehreren abgetasteten Zustandskombinationen einen Zustand aus dem ersten Zustandsraum und einen Zustand aus dem zweiten Zustandsraum aufweist,
   -- wobei der dritte Zustandsraum die Menge an mehreren abgetasteten Zustandskombinationen aufweist,
   -- wobei die ersten physiologischen Zustände und die zweiten physiologischen Zustände aus der folgenden Liste ausgewählt werden:
      --- mehrere Atemzustände einer Atmung des Patienten,
      --- mehrere Kontrastmittelzustände einer Kontrastmittelanreicherung im Patienten,
      --- mehrere kardiovaskuläre Zustände eines Herzens des Patienten,
      --- mehrere morphologische Zustände eines Tumors des Patienten,
      --- mehrere funktionelle Zustände eines Organs des Patienten,
      --- mehrere Gelenkzustände einer Extremität des Patienten,
- Festlegen einer virtuellen Zustandskombination durch einen Nutzer,
   -- wobei die virtuelle Zustandskombination nicht Teil der medizinischen Messdaten ist,
   -- wobei die virtuelle Zustandskombination einen Zustand aus dem ersten Zustandsraum und einen Zustand aus dem zweiten Zustandsraum aufweist,
   -- wobei der dritte Zustandsraum die virtuelle Zustandskombination aufweist und
   -- wobei die virtuelle Zustandskombination innerhalb des dritten Zustandsraums außerhalb der Menge an mehreren abgetasteten Zustandskombinationen liegt,
- Erzeugen eines medizinischen Bildes des Patienten,
   -- wobei ein Deformationsfeld unter Verwendung der medizinischen Messdaten ermittelt wird, wobei die durch den Nutzer festgelegte virtuelle Zustandskombination beim Ermitteln des Deformationsfelds als Eingangsparameter eingesetzt wird, wobei ein Registrieren der medizinischen Messdaten das Ermitteln des Deformationsfeldes ermöglicht, wobei das Deformationsfeld jeweils paarweise für die Menge der mehreren abgetasteten Zustandskombinationen und/oder für eine interpolierte Zustandskombination aus der Menge der mehreren abgetasteten Zustandskombinationen ermittelt wird, wobei das Deformationsfeld einer Interpolation oder einer Extrapolation der medizinischen Messdaten entspricht und wobei die medizinischen Messdaten derart durch ein Anwenden des Deformationsfelds auf die medizinischen Messdaten verformt werden, dass das erzeugte medizinische Bild die virtuelle Zustandskombination aufweist, und
- Bereitstellen des erzeugten medizinischen Bildes des Patienten.

Das Erfassen der medizinischen Messdaten kann ein Durchführen einer bildgebenden Messsequenz in einer medizinischen Bildgebungsmodalität umfassen. Typischerweise ist der Patient während der bildgebenden Messsequenz in der medizinischen Bildgebungsmodalität beispielsweise auf einer Patientenliege gelagert oder bei der medizinischen Bildgebungsmodalität positioniert. Die medizinische Bildgebungsmodalität weist einen Computertomographen (CT) auf. Die medizinischen Messdaten werden mittels des Computertomographen.

Die medizinischen Messdaten können beispielsweise Rohdaten, insbesondere Projektionsdaten des Computertomographen, und/oder mehrere Bilddatenvorlagen aufweisen. Die Rohdaten und die mehreren Bilddatenvorlagen unterscheiden sich üblicherweise in einem Speichervolumen und in einem Bildformat. Typischerweise ist das Speichervolumen der Rohdaten größer als das Speichervolumen der mehreren Bilddatenvorlagen. Die medizinischen Messdaten, insbesondere die mehreren Bilddatenvorlagen, können beispielsweise in einem DICOM-Bildformat (Digital Imaging and Communications in Medicine) vorliegen. Beispielsweise können unter Verwendung der Rohdaten, insbesondere der Projektionsdaten des Computertomographen, die mehreren Bilddatenvorlagen rekonstruiert werden, wobei typischerweise die mehreren Bilddatenvorlagen im DICOM-Bildformat vorliegen.

Vorzugsweise können aus den Projektionsdaten des Computertomographen die mehreren Bilddatenvorlagen berechnet werden, wobei in diesem Fall die mehreren Bilddatenvorlagen zumindest ein Computertomographie-Bild aufweisen. Üblicherweise weist das zumindest eine Computertomographie-Bild eine Verteilung von Bildwerten, insbesondere eine Hounsfield-Unit-Verteilung (HU), auf. Das Verfahren für das Bereitstellen des medizinischen Bildes, insbesondere eines medizinischen Computertomographie-Bildes, des Patienten ist insbesondere mittels des Computertomographen durchführbar.

Die medizinischen Messdaten können in ein Radiologieinformationssystem (RIS) und/oder in ein PACS-Bildarchivierungssystem (Picture Archiving and Communication System) übertragen und/oder abgespeichert werden. Beispielsweise können die Rohdaten und/oder die mehreren Bilddatenvorlagen in das Radiologieinformationssystem oder das PACS-Bildarchivierungssystem übertragen und/oder abgespeichert werden. Grundsätzlich ist es denkbar, dass das Erfassen der medizinischen Messdaten ein Abrufen der medizinischen Messdaten beispielsweise aus dem Radiologieinformationssystem oder dem PACS-Bildarchivierungssystem umfasst.

Typischerweise bilden ein Zustand aus dem ersten Zustandsraum und ein Zustand aus dem zweiten Zustandsraum eine Zustandskombination aus der Menge der mehreren abgetasteten Zustandskombinationen. Wenn jede der mehreren abgetasteten Zustandskombinationen einen Zustand aus dem ersten Zustandsraum und einen Zustand aus dem zweiten Zustandsraum aufweist, weist insbesondere jede der mehreren abgetasteten Zustandskombinationen zwei Zustände auf. Typischerweise entspricht die Anzahl der Dimensionen des dritten Zustandsraums einer Anzahl an Zuständen jeder Zustandskombination aus der Menge der mehreren abgetasteten Zustandskombinationen. Wenn der dritte Zustandsraum beispielsweise mehr als zwei Dimensionen aufweist, weist typischerweise jede der mehreren abgetasteten Zustandskombinationen ebenfalls mehr als zwei Dimensionen auf.

Die ersten physiologischen Zustände und die zweiten physiologischen Zustände können nach Intensität, zeitlichem Auftreten, medizinischer Relevanz, etc. vorzugsweise aufsteigend oder absteigend sortiert oder gemischt werden. Die ersten physiologischen Zustände und/oder die zweiten physiologischen Zustände können beispielsweise regelmäßig, insbesondere zyklisch, auftreten. Die ersten physiologischen Zustände und/oder die zweiten physiologischen Zustände bilden insbesondere jeweils eine, insbesondere eindimensionale, Menge oder eine, insbesondere eindimensionale, Liste, wodurch der erste Zustandsraum bzw. der zweite Zustandsraum festgelegt, insbesondere aufgespannt, wird. Grundsätzlich ist es denkbar, dass die ersten physiologischen Zustände und/oder die zweiten physiologischen Zustände jeweils eine mehrdimensionale Menge bzw. eine mehrdimensionale Liste bilden, wodurch entsprechend jeweils ein mehrdimensionaler erster Zustandsraum bzw. ein mehrdimensionaler zweiter Zustandsraum festgelegt sein kann. Üblicherweise unterscheiden sich die ersten physiologischen Zustände von den zweiten physiologischen Zuständen derart, dass der dritte Zustandsraum aufgespannt werden kann. In anderen Worten liegt der erste Zustandsraum zumindest teilweise außerhalb des zweiten Zustandsraums und umgekehrt. Der erste Zustandsraum und der zweite Zustandsraum bilden insbesondere zwei Raumachsen des dritten Zustandsraums, wobei vorzugsweise ein Winkel zwischen den Raumachsen größer als 0°, besonders vorteilhafterweise gleich 90°, ist.

Der erste Zustandsraum und der zweite Zustandsraum weisen jeweils insbesondere solche Zustände auf, welche jeweils einer Kategorie an Zuständen, zugewiesen werden kann. Wenn der erste Zustandsraum die ersten physiologischen Zustände und der zweite Zustandsraum die zweiten physiologischen Zustände aufweist, weist der erste Zustandsraum und der zweite Zustandsraum typischerweise jeweils eine Dimension auf. Sprich der erste Zustandsraum ist insbesondere eindimensional und der zweite Zustandsraum ist insbesondere eindimensional. Der erste Zustandsraum und/oder der zweite Zustandsraum und/oder der dritte Zustandsraum können diskret oder kontinuierlich sein. Typischerweise sind der erste Zustandsraum und/oder der zweite Zustandsraum vor dem Erfassen und/oder während des Erfassens der medizinischen Messdaten diskret.

Die Dimensionalität des ersten Zustandsraums, des zweiten Zustandsraums und/oder des dritten Zustandsraums ist nicht auf die obigen Ausführungen begrenzt. Durch den eindimensionalen ersten Zustandsraum und den eindimensionalen zweiten Zustandsraum wird insbesondere ein zweidimensionaler dritter Zustandsraum aufgespannt. Der dritte Zustandsraum kann mehrere Dimensionen aufweisen, wobei eine Anzahl an Dimensionen größer ist als 1. Beispielsweise ist es denkbar, dass der erste Zustandsraum eine Dimension, der zweite Zustandsraum zwei Dimensionen und damit der dritte Zustandsraum drei Dimensionen aufweist. Alternativ oder zusätzlich ist es denkbar, dass der erste Zustandsraum, der zweite Zustandsraum und ein weiterer Zustandsraum gemeinsam einen in diesem Fall typischerweise zumindest dreidimensionalen dritten Zustandsraum aufspannen. Die obigen und nachfolgenden Ausführungen über den zweidimensionalen dritten Zustandsraum stehen stellvertretend für die Ausführungsbeispiele, wobei der zumindest dritte Zustandsraum mehr als 2 Dimensionen aufweist.

Der erste Zustandsraum und/oder der zweite Zustandsraum können beispielsweise vor dem Erfassen, während dem Erfassen und/oder nach dem Erfassen der medizinischen Messdaten festgelegt und/oder ermittelt werden. Beispielsweise kann ein Nutzer vor dem Erfassen der medizinischen Messdaten einen Messparameter der bildgebenden Messsequenz derart einstellen, dass der erste Zustandsraum vorzugsweise die ersten physiologischen Zustände und/oder der zweite Zustandsraum vorzugsweise die zweiten physiologischen Zustände aufweist. Der Messparameter kann beispielweise Bezeichnungen der ersten physiologischen Zustände bzw. der zweiten physiologischen Zustände aufweisen. In diesem Fall weisen typischerweise die gemäß der bildgebenden Messsequenz erfassten medizinischen Messdaten die ersten physiologischen Zustände und/oder die zweiten physiologischen Zustände auf.

Grundsätzlich ist es denkbar, dass der Nutzer vor dem Erfassen der medizinischen Messdaten, insbesondere vor dem Abrufen der medizinischen Messdaten aus dem Radiologieinformationssystem und/oder dem PACS-Bildarchivierungssystem umfasst, den ersten Zustandsraum und/oder den zweiten Zustandsraum mittels eines Zustandsfilters bereitstellt. Der Zustandsfilter kann beispielsweise einen oder mehrere Zustände aus dem ersten Zustandsraum und/oder aus dem zweiten Zustandsraum aufweisen.

Der erste Zustandsraum und/oder der zweite Zustandsraum können beispielsweise vor dem Erfassen der medizinischen Messdaten durch den Nutzer ein erstes Mal festgelegt werden und nach dem Erfassen der medizinischen Messdaten durch den Nutzer ein zweites Mal festgelegt werden. In diesem Fall kann beispielsweise der Nutzer nach dem Erfassen der medizinischen Messdaten den ersten Zustandsraum und/oder den zweiten Zustandsraum um weitere jeweilige Zustände erweitern und/oder bestimmte Zustände ersetzen. Der Nutzer kann beispielsweise jeweils Zustände aus dem ersten Zustandsraum und/oder dem zweiten Zustandsraum zusammenfassen und/oder gewichten und/oder modellieren. Das Gewichten kann insbesondere eine Mittelwertbildung, eine Medianberechnung, eine Interpolation, eine Extrapolation, eine Addition und/oder eine Subtraktion umfassen. Da typischerweise der dritte Zustandsraum von dem ersten Zustandsraum und dem zweiten Zustandsraum abhängt, wird insbesondere bei der Festlegung des ersten Zustandsraums und/oder des zweiten Zustandsraums entsprechend der dritte Zustandsraum festgelegt.

Alternativ oder zusätzlich kann beispielsweise die Steuereinheit der medizinischen Bildgebungsmodalität den durch den Nutzer festgelegten Zustandsraum automatisch überprüfen und/oder verändern, insbesondere verkleinern oder vergrößern.

Vorzugsweise ist eine Steuereinheit der medizinischen Bildgebungsmodalität ausgebildet, die bildgebende Messsequenz derart durchzuführen, dass die medizinischen Messdaten die ersten physiologischen Zustände und/oder die zweiten physiologischen Zustände aufweisen. Wenn der erste Zustandsraum vor dem Erfassen festgelegt ist, kann die Steuereinheit der medizinischen Bildgebungsmodalität vorzugsweise die bildgebende Messsequenz prospektiv steuern, insbesondere den dritten Zustandsraum selektiv abtasten. In anderen Worten kann die Steuereinheit der medizinischen Bildgebungsmodalität ermitteln, welche der ersten physiologischen Zustände und/oder welche der zweiten physiologischen Zustände bereits erfasst wurden, und/oder festlegen, welche der ersten physiologischen Zustände und/oder welche der zweiten physiologischen Zustände als nächstes erfasst werden können. Die Steuereinheit der medizinischen Bildgebungsmodalität kann beispielsweise die Patientenliege derart verfahren, dass die medizinischen Messdaten ersten physiologischen Zustände und/oder die zweiten physiologischen Zustände aufweisen. Die Steuereinheit der medizinischen Bildgebungsmodalität kann insbesondere derart verfahren, dass die bildgebende Messsequenz prospektiv gesteuert wird, insbesondere der dritte Zustandsraum selektiv abgetastet wird. Die Patientenliege kann während dem Erfassen der medizinischen Messdaten mehrmals durch ein maximales Gesichtsfeld der medizinischen Bildgebungsmodalität hin- und hergefahren werden.

Die Menge an mehreren abgetasteten Zustandskombinationen kann beispielsweise den medizinischen Messdaten prospektiv oder retrospektiv zugeordnet werden. Die prospektive Zuordnung umfasst beispielsweise ein Hinzufügen einer Zustandskombination aus der Menge der mehreren abgetasteten Zustandskombinationen während dem Erfassen der medizinischen Messdaten, insbesondere während des Durchführens der bildgebenden Messsequenz in der medizinischen Bildgebungsmodalität zu den medizinischen Messdaten. In diesem Fall wird die Menge der mehreren abgetasteten Zustandskombinationen beispielweise durch ein physiologisches Sensorsystem ermittelt.

Die retrospektive Zuordnung kann beispielsweise ein Ermitteln der Menge der mehreren abgetasteten Zustandskombinationen unter Verwendung der medizinischen Messdaten umfassen, wobei beispielsweise mittels eines Bilderkennungsalgorithmus die medizinischen Messdaten analysiert und/oder den medizinischen Messdaten die Menge an mehreren abgetasteten Zustandskombinationen gemäß dem Bilderkennungsalgorithmus zugeordnet wird.

Grundsätzlich ist es denkbar, dass eine der mehreren abgetasteten Zustandskombinationen einen Zustand, welcher prospektiv den medizinischen Messdaten zugeordnet wird, und einen weiteren Zustand aufweist, welcher retrospektiv den medizinischen Messdaten zugeordnet wird. Des Weiteren ist es denkbar, dass die medizinischen Messdaten eine Zustandskombination aus der Menge der mehreren abgetasteten Zustandskombinationen aufweist, wobei ein Zustand dieser Zustandskombination den Rohdaten zugeordnet wird, und ein weiterer Zustand dieser Zustandskombination den mehreren Bilddatenvorlagen zugeordnet wird. Beispielsweise kann die Zustandskombination aus der Menge der mehreren abgetasteten Zustandskombinationen als Teil des DICOM-Bildformats, insbesondere im Header des DICOM-Bildformats, abgespeichert sein.

Grundsätzlich ist es außerdem denkbar, dass nach dem Erfassen der medizinischen Messdaten die Menge an mehreren abgetasteten Zustandskombinationen, insbesondere die ersten physiologischen Zustände und/oder die zweiten physiologischen Zustände, unter Verwendung der medizinischen Messdaten ermittelt werden. In diesem Fall werden der erste Zustandsraum und der zweite Zustandsraum gemäß der ermittelten Menge an mehreren abgetasteten Zustandskombinationen nach dem Erfassen der medizinischen Messdaten festgelegt.

Alternativ oder zusätzlich kann beispielsweise während dem Erfassen der medizinischen Messdaten die Steuereinheit der medizinischen Bildgebungsmodalität oder eine Steuereinheit des Radiologieinformationssystems den ersten Zustandsraum, insbesondere die ersten physiologischen Zustände, und/oder den zweiten Zustandsraum, insbesondere die zweiten physiologischen Zustände, ermitteln. In diesem Fall ist beispielsweise die Steuereinheit der medizinischen Bildgebungsmodalität derart ausgebildet, die ersten physiologischen Zustände und/oder die zweiten physiologischen Zustände zu ermitteln, und den ersten Zustandsraum und/oder den zweiten Zustandsraum zu ermitteln bzw. festzulegen.

Die Menge an mehreren abgetasteten Zustandskombinationen ist insbesondere innerhalb des dritten Zustandsraums enthalten. Typischerweise kann die Menge an mehreren abgetasteten Zustandskombinationen durch den ersten Zustandsraum und durch den zweiten Zustandsraum mathematisch, beispielsweise mittels einer Mengenfunktion, beschrieben werden.

Üblicherweise können die medizinischen Messdaten in Messdatenblöcke unterteilt werden, wobei den jeweiligen Messdatenblöcken die Menge an mehreren abgetasteten Zustandskombinationen zugeordnet wird. Ein Messdatenblock kann beispielsweise diejenigen medizinischen Messdaten aufweisen, welche während eines Zeitintervalls erfasst worden sind. Typischerweise kann das Zeitintervall mit einer Zeitauflösung der bildgebenden Messsequenz korrelieren oder der Zeitauflösung der bildgebenden Messsequenz entsprechen. Alternativ oder zusätzlich kann der Messdatenblock diejenigen medizinischen Messdaten aufweisen, welche einen Teil, insbesondere einen räumlichen Abschnitt, eines Messbereichs der medizinischen Bildgebungsmodalität aufweisen und/oder abbilden. Beispielsweise kann der räumliche Abschnitt eine z-Position des Messbereichs der medizinischen Bildgebungsmodalität und/oder eine Ausdehnung des Messbereichs entlang der z-Achse der medizinischen Bildgebungsmodalität aufweisen. Die z-Achse der medizinischen Bildgebungsmodalität ist typischerweise parallel zur Patientenliege ausgerichtet. Die Messdatenblöcke können jeweils eine Zustandskombination aus der Menge der mehreren abgetasteten Zustandskombinationen aufweisen.

Das medizinische Bild des Patienten wird insbesondere unter Verwendung der medizinischen Messdaten rekonstruiert. Typischerweise wird das medizinische Bild des Patienten in dem DICOM-Bildformat gespeichert. Vorzugsweise kann das medizinische Bild des Patienten in das Radiologieinformationssystem und/oder das PACS-Bildarchivierungssystem übertragen werden. Das Erzeugen des medizinischen Bildes umfasst insbesondere ein Ermitteln des medizinischen Bildes unter Verwendung der medizinischen Messdaten.

Die virtuelle Zustandskombination kann üblicherweise beim Erzeugen des medizinischen Bildes dem medizinischen Bild zugeordnet werden. Die virtuelle Zustandskombination unterscheidet sich insbesondere von der Menge an mehreren abgetasteten Zustandskombinationen derart, dass die medizinischen Messdaten lediglich die Menge an mehreren abgetasteten Zustandskombinationen aufweist. Die virtuelle Zustandskombination ist nicht Teil der medizinischen Messdaten.
Typischerweise werden die medizinischen Messdaten in Abhängigkeit von der Menge an mehreren medizinischen Zustandskombinationen erfasst und/oder abgetastet. Üblicherweise kann die Menge an mehreren medizinischen Zustandskombinationen den medizinischen Messdaten zugeordnet werden.

Typischerweise unterscheidet sich also die virtuelle Zustandskombination von einer abgetasteten Zustandskombination darin, dass lediglich für die Menge an mehreren abgetasteten Zustandskombinationen die medizinischen Messdaten erfasst werden, wobei die Menge an mehreren abgetasteten Zustandskombinationen die virtuelle Zustandskombination nicht aufweist. Die virtuelle Zustandskombination wird insbesondere unabhängig von den medizinischen Messdaten und/oder von der Menge an mehreren abgetasteten Zustandskombinationen, durch den Nutzer festgelegt.

Die virtuelle Zustandskombination ist insbesondere innerhalb des dritten Zustandsraums enthalten. Vorzugsweise kann die virtuelle Zustandskombination durch den ersten Zustandsraum und durch den zweiten Zustandsraum mathematisch, beispielsweise mittels einer weiteren Mengenfunktion, beschrieben werden.

Die virtuelle Zustandskombination kann einen Zustand aus dem ersten Zustandsraum und/oder einen Zustand aus dem zweiten Zustandsraum aufweisen. Alternativ oder zusätzlich kann die virtuelle Zustandskombination einen weiteren Zustand, welcher dem ersten Zustandsraum zugeordnet wird und den ersten Zustandsraum erweitern kann, und/oder einen weiteren Zustand, welcher dem zweiten Zustandsraum zugeordnet wird und den zweiten Zustandsraum erweitern kann, aufweisen. Beispielsweise ist es denkbar, dass der weitere Zustand aus dem ersten Zustandsraum Anteile von mehreren Zuständen aus dem ersten Zustandsraum und/oder Anteile von allen Zuständen aus dem ersten Zustandsraum aufweist. Beispielsweise kann der weitere Zustand aus dem ersten Zustandsraum derart berechnet sein, dass der weitere Zustand einem Mischzustand gemäß den Anteilen der mehreren Zustände aus dem ersten Zustandsraum und/oder aller Zustände aus dem ersten Zustandsraum entspricht. In anderen Worten kann der weitere Zustand durch Gewichtung, insbesondere durch Mittelwertbildung, der Anteile der mehreren Zustände aus dem ersten Zustandsraum und/oder aller Zustände aus dem ersten Zustandsraum berechnet sein. Grundsätzlich kann jeder Zustandsraum, insbesondere der erste Zustandsraum, der zweite Zustandsraum und/oder der dritte Zustandsraum, den Mischzustand aufweisen. In diesem Fall kann ein üblicherweise diskreter erster Zustandsraum und/oder ein üblicherweise diskreter zweiter Zustandsraum durch das Hinzufügen des Mischzustands in einen kontinuierlichen ersten Zustandsraum bzw. in einen kontinuierlichen zweiten Zustandsraum transformiert werden, wodurch üblicherweise der dritte Zustandsraum ebenfalls kontinuierlich wird.

Eine Schnittmenge zwischen der virtuellen Zustandskombination und der Menge an mehreren abgetasteten Zustandskombination ist typischerweise gleich null. Die virtuelle Zustandskombination kann typischerweise dem medizinischen Bild zugeordnet werden, aber nicht den medizinischen Messdaten. In anderen Worten ist in einer Liste der Menge an mehreren abgetasteten Zustandskombinationen die virtuelle Zustandskombination nicht enthalten. Vorzugsweise ist ein Überlappen zwischen der virtuellen Zustandskombination und der Menge an mehreren abgetasteten Zustandskombinationen unmöglich. Typischerweise liegen die virtuelle Zustandskombination und die Menge an mehreren abgetasteten Zustandskombinationen innerhalb des dritten Zustandsraums nebeneinander. Typischerweise ist ein Zustand der virtuellen Zustandskombination relativ zu der Menge an mehreren abgetasteten Zustandskombinationen entlang einer Dimension, insbesondere einer Raumachse, des dritten Zustandsraums verschoben.

Das Bereitstellen des medizinischen Bildes kann ein Übertragen des medizinischen Bildes in das Radiologieinformationssystem und/oder das PACS-Bildarchivierungssystem umfassen. Alternativ oder zusätzlich kann das medizinische Bild, insbesondere dem Nutzer, auf einem Monitor angezeigt werden. Vorzugsweise ist der Monitor, beispielsweise zu dem Abrufen des medizinischen Bildes aus dem Radiologieinformationssystem bzw. dem PACS-Bildarchivierungssystem und/oder aus der Steuereinheit der medizinischen Bildgebungsmodalität verbunden, und/oder zu einem Anzeigen des medizinischen Bildes ausgebildet. Grundsätzlich ist es denkbar, dass das medizinische Bild über ein Netzwerk, welches beispielsweise das Radiologieinformationssystem und/oder das PACS-Bildarchivierungssystem und/oder die medizinische Bildgebungsmodalität verbindet, ausgetauscht und beispielsweise auf einem Server gespeichert bzw. abgerufen werden kann. Zusätzlich zu dem medizinischen Bild können beispielsweise die medizinischen Messdaten bereitgestellt, insbesondere in das Radiologieinformationssystem und/oder das PACS-Bildarchivierungssystem übertragen und/oder auf dem Monitor angezeigt werden.

Das erfindungsgemäße Verfahren für das Bereitstellen des medizinischen Bildes des Patienten bringt insbesondere folgende Vorteile mit sich:
Das Erzeugen des medizinischen Bildes für quasi beliebige Zustandskombinationen ist insbesondere in Hinblick einer Reduzierung von einer Untersuchungsdauer des Patienten vorteilhaft. Des Weiteren korreliert die Untersuchungsdauer des Patienten typischerweise beim Durchführen der bildgebenden Messsequenz in dem Computertomographen und/oder in dem konventionellen Röntgensystem mit einer potentiell für den Patienten schädlichen Röntgenstrahlung. Durch das vorteilhafte prospektive Steuern der bildgebenden Messsequenz, insbesondere das selektive Abtasten des dritten Zustandsraums, kann die Untersuchungsdauer und vorzugsweise die mit der Röntgenstrahlung korrespondierende Röntgendosis verringert werden. Besonders vorteilhaft ist, wenn insbesondere lediglich der erste Zustandsraum und/oder der zweite Zustandsraum für sich genommen vollständig in den medizinischen Messdaten abgebildet werden.

Die Menge der mehreren abgetasteten Zustandskombinationen ist vorzugsweise nur so groß, dass der erste Zustandsraum und der zweite Zustandsraum in den medizinischen Messdaten abgebildet ist.

Das erfindungsgemäße Verfahren ermöglicht insbesondere ein Bereitstellen des medizinischen Bildes für eine Zustandskombination des dritten Zustandsraums unabhängig davon, ob für diese Zustandskombinationen die medizinischen Messdaten erfasst werden. Dies ist insbesondere für den Nutzer vorteilhaft, wenn der Nutzer nach dem Erfassen der medizinischen Messdaten das medizinische Bild für eine spezifische Zustandskombination erzeugen und bereitstellen kann, für welche keine medizinischen Messdaten erfasst werden. Ein weiterer Vorteil kann ein Ersetzen der medizinischen Messdaten für die Zustandskombinationen sein, für welche beispielsweise das Erfassen der medizinischen Messdaten technisch nicht möglich ist oder aufgrund unzureichender Datenqualität der medizinischen Messdaten dieselben erneut erfasst werden müssten.

Eine Ausführungsform sieht vor, dass das medizinische Bild des Patienten derart unter Verwendung der medizinischen Messdaten erzeugt wird, dass die virtuelle Zustandskombination und eine Zustandskombination aus der Menge der mehreren abgetasteten Zustandskombinationen denselben Zustand aus dem ersten Zustandsraum und/oder denselben Zustand aus dem zweiten Zustandsraum aufweisen. In anderen Worten ist in diesem Ausführungsbeispiel ein Zustand der virtuellen Zustandskombination relativ zu der Menge an mehreren abgetasteten Zustandskombinationen entlang lediglich einer Dimension des dritten Zustandsraums verschoben. Vorzugsweise kann der verschobene Zustand der virtuellen Zustandskombination einfach ermittelt werden. Typischerweise ist in dieser Ausführungsform die virtuelle Zustandskombination auf diejenigen Zustandskombinationen eingeschränkt, welche durch die ersten physiologischen Zustände des ersten Zustandsraums und durch die zweiten physiologischen Zustände des zweiten Zustandsraums vorgegeben sind. In anderen Worten kann in dieser Ausführungsform der dritte Zustandsraum diskret sein.

Eine Ausführungsform sieht vor, dass die medizinischen Messdaten solange erfasst werden, bis die medizinischen Messdaten jeden Zustand aus dem ersten Zustandsraum und/oder jeden Zustand aus dem zweiten Zustandsraum einmal aufweisen. Diese Ausführungsform beschreibt ein Beispiel des selektiven Abtastens des dritten Zustandsraums. Beispielsweise kann die Steuereinheit der medizinischen Bildgebungsmodalität zu einem Ermitteln der Zustände aus dem ersten Zustandsraum und/oder der Zustände aus dem zweiten Zustandsraum ausgebildet sein, wobei die Steuereinheit vorzugsweise das Durchführen der bildgebenden Messsequenz beendet, sobald die medizinischen Messdaten jeden Zustand aus dem ersten Zustandsraum und/oder jeden Zustand aus dem zweiten Zustandsraum einmal aufweisen. In anderen Worten wird die bildgebende Messsequenz derart gesteuert, dass die medizinischen Messdaten jeden Zustand aus dem ersten Zustandsraum und/oder jeden Zustand aus dem zweiten Zustandsraum mindestens einmal aufweisen.

Alternativ oder zusätzlich kann beim Erfassen der medizinischen Messdaten, wobei die medizinischen Messdaten aus dem Radiologieinformationssystem oder aus dem PACS-Bildarchivierungssystem abgerufen werden, der Nutzer das Abrufen der medizinischen Messdaten beenden, sobald die medizinischen Messdaten jeden Zustand aus dem ersten Zustandsraum und/oder jeden Zustand aus dem zweiten Zustandsraum einmal aufweisen. Dies ist insbesondere in diesem Fall vorteilhaft, wenn der Nutzer gemäß einer medizinischen Fragestellung den ersten Zustandsraum und/oder den zweiten Zustandsraum vor dem Erfassen und/oder nach dem Erfassen der medizinischen Messdaten festlegt.

Eine Ausführungsform sieht vor, dass die medizinischen Messdaten solange erfasst werden, bis die medizinischen Messdaten jeden Zustand aus dem ersten Zustandsraum und jeden Zustand aus dem zweiten Zustandsraum lediglich einmal aufweisen. Beispielsweise ist die Steuereinheit der medizinischen Bildgebungsmodalität derart ausgebildet, dass die medizinischen Messdaten nur solange erfasst werden, bis die medizinischen Messdaten jeden Zustand aus dem ersten Zustandsraum und jeden Zustand aus dem zweiten Zustandsraum lediglich einmal aufweisen. Dieses Ausführungsbeispiel ist in Hinblick auf eine minimale Untersuchungsdauer, insbesondere minimale Röntgenstrahlendosis, besonders vorteilhaft. Diese Ausführungsform beschreibt ein besonders bevorzugtes Beispiel des selektiven Abtastens des dritten Zustandsraums.

Die mehreren Atemzustände sind typischerweise zyklisch und weisen beispielsweise die Zustände Einatmen, mittleres Atmen und/oder Ausatmen auf. Die mehreren Kontrastmittelzustände weisen beispielsweise die Zustände keine Kontrastmittelanreicherung, ansteigende Kontrastmittelanreicherung, maximale Kontrastmittelanreicherung und/oder absteigende Kontrastmittelanreicherung auf. Üblicherweise werden die mehreren Kontrastmittelzustände durch eine Injektion eines Kontrastmittelbolus in den Patienten ermöglicht. Die kardiovaskulären Zustände des Herzens sind typischerweise zyklisch und weisen insbesondere systolische und diastolische Herzphasen auf. Die morphologischen Zustände des Tumors weisen insbesondere Neovaskularisation, Tumorwachstum und Nekrose auf. Die funktionellen Zustände des Organs können beispielsweise einen Füllstand einer Blase des Patienten aufweisen (von leer bis voll). Die mehreren Gelenkzustände der Extremität des Patienten weisen insbesondere einen ausgestreckten oder angewinkelten Arm auf.

In einem Beispiel kann der Mischzustand beispielsweise die mehreren Atemphasen aufweisen, wobei der Mischzustand vorzugsweise gemäß den mehreren Atemphasen berechnet und/oder gemittelt ist.

Das physiologische Sensorsystem kann beispielsweise einen Atemgurt, einen Echokardiographen, eine Kontrastmittelpumpe und/oder eine Kamera aufweisen. Beispielsweise können mittels des Atemgurts oder der Kamera die mehreren Atemzustände der Atmung des Patienten ermittelt werden, wobei die mehreren Atemzustände den medizinischen Messdaten zugeordnet werden können. Alternativ oder zusätzlich können mittels der Kontrastmittelpumpe und/oder mittels der medizinischen Messdaten die mehreren Kontrastmittelzustände der Kontrastmittelanreicherung des Patienten erfasst werden.

Der erste Zustandsraum kann erste morphologische Zustände des Patienten und/oder erste funktionelle Zustände des Patienten aufweisen. Wenn der erste Zustandsraum die ersten morphologischen Zustände des Patienten und die ersten funktionellen Zustände des Patienten aufweist, ist der erste Zustandsraum insbesondere zweidimensional. Alternativ oder zusätzlich können beispielsweise die mehreren Atemzustände, die mehreren Kontrastmittelzustände, die mehreren kardiovaskulären Zustände, die morphologischen Zustände des Tumors, die funktionellen Zustände des Organs und/oder die mehreren Gelenkzustände jeweils morphologische Subzustände und/oder physiologische Subzustände und/oder funktionelle Subzustände aufweisen. Beispielsweise können die mehreren Atemzustände der Atmung des Patienten erste physiologische Atemzustände und zweite morphologische Atemzustände aufweisen. In diesem Fall ist es denkbar, dass der erste Zustandsraum physiologische Atemzustände und der zweite Zustandsraum morphologische Atemzustände aufweist. Dies ist insbesondere vorteilhaft, wenn beispielsweise durch die Atmung des Patienten die Morphologie und die Physiologie des Patienten verändert werden. In diesem Fall kann sich beispielsweise beim Einatmen ein Brustkorb des Patienten heben, wodurch sich insbesondere der morphologische Atemzustand verändert, und gleichzeitig insbesondere ein Luft-Wasser-Verhältnis in einer Lunge des Patienten variiert, wodurch der physiologische Atemzustand verändert sein kann. Typischerweise wäre das Heben des Brustkorbs als ein Verschieben des Brustkorbs in den medizinischen Messdaten ersichtlich, während die Variation des Luft-Wasser-Verhältnisses durch eine Veränderung beispielsweise eines Schwächungskoeffizienten, insbesondere einer Hounsfield-Einheit, und/oder einer Relaxationszeit (T1-Zeit, T2-Zeit, T2*-Zeit) und/oder einer Protonendichte ersichtlich werden kann.

Eine Ausführungsform sieht vor, dass der erste Zustandsraum die mehreren Atemzustände der Atmung des Patienten aufweist und wobei der zweite Zustandsraum die mehreren Kontrastmittelzustände der Kontrastmittelanreicherung im Patienten aufweist. In diesem Fall weist der dritte Zustandsraum eine Raumachse über die mehreren Atemzustände und eine Raumachse über die mehreren Kontrastmittelzustände auf. Grundsätzlich ist es denkbar, dass beispielsweise der weitere Zustandsraum die kardiovaskulären Zustände des Patienten aufweist. Besonders vorteilhaft ist, dass dem Nutzer das medizinische Bild für eine beliebige virtuelle Zustandskombination der Atmung und der Kontrastmittelanreicherung bereitgestellt werden kann.

Eine Ausführungsform sieht vor, dass die Menge an mehreren abgetasteten Zustandskombinationen den dritten Zustandsraum maximal bis einschließlich 50 Prozent auffüllt. Wenn in einem Beispiel der erste Zustandsraum zwei erste physiologische Zustände und der zweite Zustandsraum zwei zweite physiologische Zustände aufweist, werden vorzugsweise die medizinischen Messdaten solange erfasst, bis die Menge an mehreren abgetasteten Zustandskombinationen vorzugsweise zwei Zustandskombinationen aufweist, wobei die medizinischen Messdaten jeden Zustand aus dem ersten Zustandsraum und jeden Zustand aus dem zweiten Zustandsraum lediglich einmal aufweisen. In diesem Beispiel füllen die virtuelle Zustandskombination und die Menge an mehreren abgetasteten Zustandskombinationen den dritten Zustandsraum vorzugsweise zu 75 Prozent auf. Typischerweise vergrößert die virtuelle Zustandskombination die Auffüllung des dritten Zustandsraums in Kombination mit der Menge an mehreren abgetasteten Zustandskombinationen. Wenn der erste Zustandsraum und/oder der zweite Zustandsraum jeweils mehr als zwei Zustände aufweisen, füllt typischerweise die Menge an mehreren abgetasteten Zustandskombinationen den dritten Zustandsraum weniger als 50 Prozent auf. Die Menge an mehreren abgetasteten Zustandskombinationen füllt den dritten Zustandsraum insbesondere weniger als 30 Prozent, vorzugsweise weniger als 20 Prozent oder besonders vorteilhafterweise weniger als 10 Prozent auf.

Eine Ausführungsform sieht vor, dass unter Verwendung der medizinischen Messdaten die mehreren Bilddatenvorlagen rekonstruiert werden, wobei das medizinische Bild unter Verwendung der mehreren Bilddatenvorlagen erzeugt wird. Üblicherweise werden zunächst unter Verwendung der Rohdaten der medizinischen Messdaten die mehreren Bilddatenvorlagen rekonstruiert und in einem weiteren Schritt das medizinische Bild erzeugt. Dies ist insbesondere vorteilhaft, weil das Speichervolumen der mehreren Bilddatenvorlagen typischerweise kleiner als das Speichervolumen der Rohdaten ist.

Eine Ausführungsform sieht vor, dass mehrere medizinische Bilder des Patienten unter Verwendung der medizinischen Messdaten erzeugt werden, wobei die mehreren medizinischen Bilder eine Menge an mehreren virtuellen Zustandskombinationen aufweisen, wobei jede der mehreren virtuellen Zustandskombinationen einen Zustand aus dem ersten Zustandsraum und einen Zustand aus dem zweiten Zustandsraum aufweist, wobei der dritte Zustandsraum die Menge an mehreren virtuellen Zustandskombinationen aufweist und wobei die Menge an mehreren virtuellen Zustandskombinationen innerhalb des dritten Zustandsraum außerhalb der Menge an mehreren abgetasteten Zustandskombinationen liegt. Vorzugsweise ist eine Schnittmenge zwischen der Menge an mehreren virtuellen Zustandskombinationen und der Menge an mehreren abgetasteten Zustandskombinationen gleich null.

Insbesondere die Menge an mehreren abgetasteten Zustandskombinationen gemeinsam mit der virtuellen Zustandskombination, insbesondere gemeinsam mit der Menge an mehreren virtuellen Zustandskombinationen, füllen den dritten Zustandsraum einschließlich bis zu 90%, besonders vorteilhafterweise bis zu 100% auf. Wenn die Auffüllung des dritten Zustandsraums 100% beträgt, ist vorzugsweise für jede Zustandskombination der ersten physiologischen Zustände und der zweiten physiologischen Zustände entweder das medizinische Bild, insbesondere die mehreren medizinischen Bilder, oder die medizinischen Messdaten, insbesondere die mehreren Bilddatenvorlagen, vorhanden.

Das Deformationsfeld kann insbesondere ein Vektorfeld aufweisen, welches eine Veränderung zwischen einer Startzustandskombination und einer Zielzustandskombination abbildet. Das Deformationsfeld kann mehrere Vektorfelder aufweisen, welche einen Pfad von der Startzustandskombination zur Zielzustandskombination beschreiben. Das Deformationsfeld entspricht einer Interpolation oder einer Extrapolation der medizinischen Messdaten. Durch das Anwenden des Deformationsfelds werden die medizinischen Messdaten, typischerweise entlang des Pfads, derart verformt, dass das erzeugte medizinische Bild die virtuelle Zustandskombination, insbesondere die Zielzustandskombination, aufweist. Vorzugsweise kann durch Inversion des Deformationsfelds die Verformung umgedreht werden.

Wenn das Deformationsfeld jeweils paarweise für die Menge der mehreren abgetasteten Zustandskombinationen ermittelt wird, kann das Deformationsfeld für jedes beliebige Paar aus der Menge der mehreren abgetasteten Zustandskombinationen das Vektorfeld aufweisen. In anderen Worten ist insbesondere jede Zustandskombination aus der Menge der mehreren abgetasteten Zustandskombinationen für jeweils ein Vektorfeld Startzustandskombination und Zielzustandskombination. Zusätzlich weist das Deformationsfeld ein virtuelles Vektorfeld auf, wobei die virtuelle Zustandskombination der Zielzustandskombination entspricht und eine Zustandskombination aus der Menge der mehreren abgetasteten Zustandskombinationen der Startzustandskombination entspricht. Das virtuelle Vektorfeld beschreibt insbesondere einen Schritt der Verformung der medizinischen Messdaten zum medizinischen Bild. In diesem Fall umfasst das Erzeugen des medizinischen Bildes insbesondere ein Verketten der Vektorfelder innerhalb der Menge der mehreren abgetasteten Zustandskombinationen und des virtuellen Vektorfeldes. Das Erzeugen des medizinischen Bildes weist insbesondere zumindest das Anwenden des virtuellen Vektorfeldes auf die medizinischen Messdaten auf.

Wenn das Deformationsfeld für die interpolierte Zustandskombination aus der Menge der mehreren abgetasteten Zustandskombinationen ermittelt wird, beschreibt das virtuelle Vektorfeld ein Verformen zwischen der interpolierten Zustandskombination als Startzustandskombination und der virtuellen Zustandskombination als Zielzustandskombination. In diesem Fall ist die interpolierte Zustandskombination die Zielzustandskombination für jede Zustandskombination aus der Menge der mehreren abgetasteten Zustandskombinationen. Die interpolierte Zustandskombination liegt vorzugsweise innerhalb des dritten Zustandsraums außerhalb der Menge an mehreren abgetasteten Zustandskombinationen, besonders vorteilhafterweise zwischen den ersten physiologischen Zuständen und den zweiten physiologischen Zuständen. Die interpolierte Zustandskombination ermöglicht insbesondere ein kontinuierliches Abtasten des dritten Zustandsraums. In anderen Worten erfordert der interpolierte Zustandsraum, dass der dritte Zustandsraum kontinuierlich ist. In einer besonders bevorzugten Ausführung entspricht die interpolierte Zustandskombination einer gemittelten Zustandskombination. Wenn der erste Zustandsraum die beiden Zustände Einatmen und Ausatmen aufweist und der zweite Zustandsraum die beiden Zustände keine Kontrastmittelanreicherung und volle Kontrastmittelanreicherung aufweist, dann entspricht die gemittelte Zustandskombination beispielsweise dem mittleren Atmen und der ansteigenden Kontrastmittelanreicherung des Patienten. Weiterhin ist besonders vorteilhaft, dass das medizinische Bild lediglich durch das Anwenden zweier Vektorfelder, wobei eines davon dem virtuellen Vektorfeld entspricht, erzeugt werden kann.

Das Modellieren und/oder das Registrieren der medizinischen Messdaten kann für den ersten Zustandsraum, den zweiten Zustandsraum und/oder den dritten Zustandsraum separat, teilweise oder allgemein erfolgen. Das Modellieren und/oder das Registrieren der medizinischen Messdaten kann ein Verwenden eines physiologischen Modells, eines strukturellen Modells, eines anatomischen Modells und/oder eines morphologischen Modells aufweisen. Das Modellieren kann Randbedingungen und/oder Fitten von mathematischen Funktionen erfordern. Das Modellieren der medizinischen Messdaten kann eine zyklische Randbedingung, beispielsweise wie bei den mehreren Atemphasen, aufweisen. Alternativ oder zusätzlich kann ein Modell der Kontrastmittelanreicherung, beispielsweise eine Gamma-Variate-Funktion, an die medizinischen Messdaten gefittet werden. Das Modellieren der medizinischen Messdaten ermöglicht insbesondere das kontinuierliche Abtasten des dritten Zustandsraums. Das Registrieren der medizinischen Messdaten ermöglicht ein Ermitteln des Deformationsfeldes, insbesondere der Vektorfelder.

Eine Ausführungsform sieht vor, dass das Erzeugen des medizinischen Bildes weitere folgende Schritte umfasst: - Aufteilen der medizinischen Messdaten in einen niederfrequenten Messdatenanteil und in einen hochfrequenten Messdatenanteil nach dem Anwenden des Deformationsfelds und - Kombinieren des niederfrequenten Messdatenanteils und des deformierten Messdatenanteils mit unterschiedlichen Gewichtungen, wodurch das medizinische Bild erzeugt wird.

Üblicherweise ergeben der niederfrequente Messdatenanteil und der hochfrequente Messdatenanteil in Kombination die medizinischen Messdaten. Typischerweise weist der hochfrequente Messdatenanteil Informationen über Kanten und Bildrauschen auf, während der niederfrequente Messdatenanteil Informationen mit niedriger Raumfrequenz aufweist. Grundsätzlich ist es denkbar, dass das Deformationsfeld auf den hochfrequenten Messdatenanteil und auf den niederfrequenten Messdatenanteil separat angewendet wird oder dass das Deformationsfeld auf die medizinischen Messdaten vor dem Aufteilen angewendet wird. Vorzugsweise unterscheidet sich die Gewichtung bei der Kombination des niederfrequenten Messdatenanteils von der Gewichtung bei der Kombination des hochfrequenten Messdatenanteils. Beispielsweise kann der hochfrequente Messdatenanteil gemittelt und der niederfrequente Messdatenanteil durch konvexe Kombination gewichtet werden. Des Weiteren ist es denkbar, dass die Gewichtung lokal, beispielsweise abhängig von einem Organ des Patienten, oder global festgelegt wird. Beispielsweise kann der niederfrequente Messdatenanteil einer Lunge des Patienten anders gewichtet werden als der niederfrequente Messdatenanteil einer Leber des Patienten. Alternativ oder zusätzlich kann die Gewichtung von einer Bildintensität der medizinischen Messdaten, insbesondere der HU-Verteilung, abhängen.

Die erfindungsgemäße Anlage für ein Bereitstellen eines medizinischen Bildes eines Patienten weist eine Recheneinheit auf. Die Recheneinheit ist insbesondere zu einem Ausführen der Steuereinheit der medizinischen Bildgebungsmodalität ausgebildet, insbesondere wenn die Steuereinheit der medizinischen Bildgebungsmodalität in Programmcodemitteln abgebildet ist. Die Recheneinheit kann beispielsweise mittels des Netzwerks mit dem Radiologieinformationssystem und/oder dem PACS-Bildarchivierungssystem zu dem Erfassen der medizinischen Messdaten und/oder zu dem Bereitstellen des medizinischen Bildes verbunden sein. Alternativ oder zusätzlich kann die Anlage für das Bereitstellen des medizinischen Bildes des Patienten die medizinische Bildgebungsmodalität und/oder den Monitor aufweisen. In diesem Fall ist die Recheneinheit vorzugsweise mit der medizinischen Bildgebungsmodalität derart verbunden, dass die medizinischen Messdaten, insbesondere die Rohdaten, von der medizinischen Bildgebungsmodalität zur Recheneinheit übertragen werden können.

Die Anlage weist einen Computertomographen auf, welcher zum Erfassen der medizinischen Messdaten des Patienten ausgebildet ist. In diesem Beispiel weist die Anlage für das Bereitstellen des medizinischen Bildes des Patienten die medizinische Bildgebungsmodalität, insbesondere den Computertomographen, auf. Der Computertomograph weist insbesondere einen Röntgenstrahler und einen Röntgendetektor auf, welche drehbar um die Patientenliege gelagert sind. Vorzugsweise ist die Steuereinheit der medizinischen Bildgebungsmodalität zum Steuern des Computertomographen, insbesondere der Patientenliege, ausgebildet.

Das erfindungsgemäße Computerprogrammprodukt weist Programmcodemittel auf, welche in die Recheneinheit ladbar sind, um ein Verfahren für ein Bereitstellen eines medizinischen Bildes eines Patienten auszuführen, wenn die Programmcodemittel in der Recheneinheit ausgeführt werden.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Flussdiagramm eines Verfahrens für ein Bereitstellen eines medizinischen Bildes eines Patienten in einem ersten Ausführungsbeispiel,
Fig. 2 eine Anlage für ein Bereitstellen eines medizinischen Bildes eines Patienten,
Fig. 3 eine schematische Abbildung eines dritten Zustandsraumes in einem zweiten Ausführungsbeispiel,
Fig. 4 eine schematische Abbildung eines dritten Zustandsraumes in einem dritten Ausführungsbeispiel und
Fig. 5 ein Flussdiagramm des Verfahrens in einem vierten Ausführungsbeispiel.

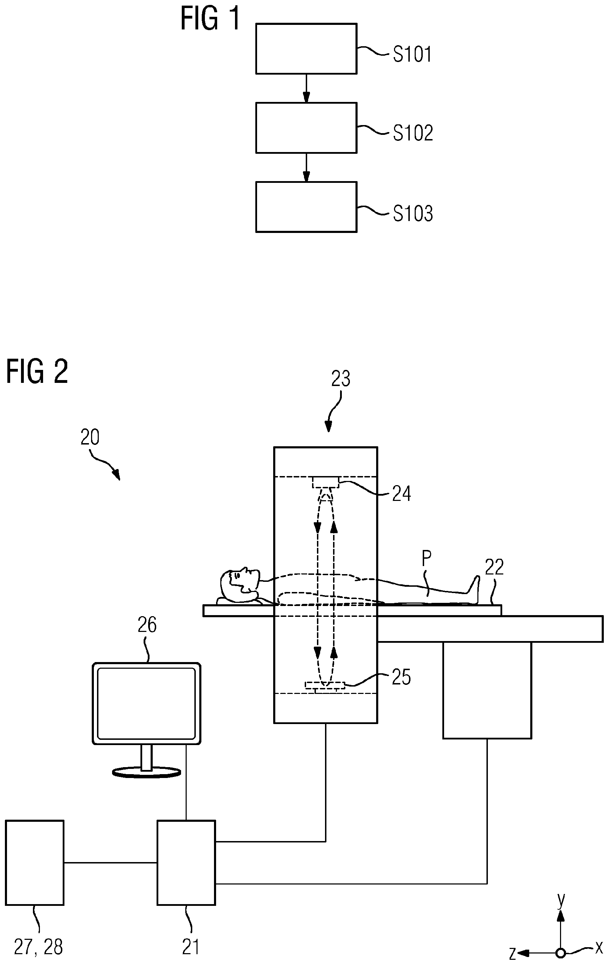

**Fig. 1** zeigt ein Flussdiagramm eines Verfahrens für ein Bereitstellen eines medizinischen Bildes eines Patienten in einem ersten Ausführungsbeispiel.

Verfahrensschritt S101 kennzeichnet das Erfassen von medizinischen Messdaten des Patienten, wobei die medizinischen Messdaten eine Menge an mehreren abgetasteten Zustandskombinationen aufweisen, wobei ein erster Zustandsraum erste physiologische Zustände des Patienten aufweist, wobei ein zweiter Zustandsraum zweite physiologische Zustände des Patienten aufweist und wobei der erste Zustandsraum und der zweite Zustandsraum zusammen einen dritten Zustandsraum aufspannen, wobei jede der mehreren abgetasteten Zustandskombinationen einen Zustand aus dem ersten Zustandsraum und einen Zustand aus dem zweiten Zustandsraum aufweist und wobei der dritte Zustandsraum die Menge an mehreren abgetasteten Zustandskombinationen aufweist.

Verfahrensschritt S102 kennzeichnet das Erzeugen eines medizinischen Bildes des Patienten unter Verwendung der medizinischen Messdaten, wobei das medizinische Bild eine virtuelle Zustandskombination aufweist, wobei die virtuelle Zustandskombination einen Zustand aus dem ersten Zustandsraum und einen Zustand aus dem zweiten Zustandsraum aufweist, wobei der dritte Zustandsraum die virtuelle Zustandskombination aufweist und wobei die virtuelle Zustandskombination innerhalb des dritten Zustandsraums außerhalb der Menge an mehreren abgetasteten Zustandskombinationen liegt.

Verfahrensschritt S103 kennzeichnet das Bereitstellen des medizinischen Bildes des Patienten.

**Fig. 2** zeigt die Anlage 20 für ein Bereitstellen des medizinischen Bildes des Patienten P, wobei die Anlage 20 eine Recheneinheit 21 aufweist. Der Patient P ist auf einer Patientenliege 22 gelagert, wobei die Patientenliege 22 parallel zur z-Achse ausgebildet ist. Die Recheneinheit 21 weist einen Monitor 26 auf, welcher beispielsweise eine graphische Benutzeroberfläche sowie Eingabemittel aufweist. Die Recheneinheit 21 ist mit einem Radiologieinformationssystem 27 und einem PACS-Bildarchivierungssystem 28 verbunden.

Die Anlage 20 weist einen Computertomographen 23 auf, welcher zum Erfassen der medizinischen Messdaten des Patienten P ausgebildet ist. Der Computertomograph 23 weist einen Röntgenstrahler 24 und einen Röntgendetektor 25 auf, wobei der Röntgenstrahler 24 und der Röntgendetektor 25 um die Patientenliege 22 drehbar angeordnet sind.

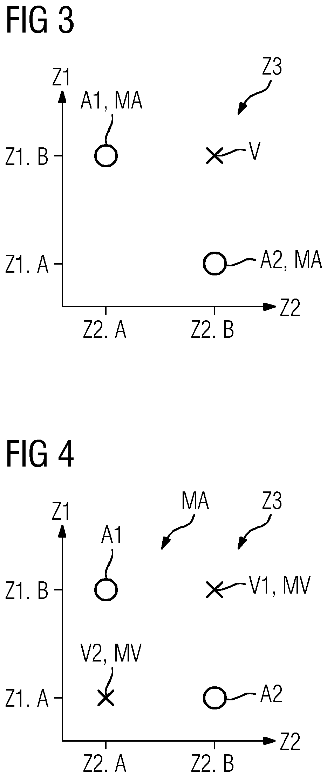

**Fig. 3** zeigt eine schematische Abbildung des dritten Zustandsraums Z3 in einem zweiten Ausführungsbeispiel. Der erste Zustandsraum Z1 weist die ersten physiologischen Zustände Z1.A und Z1.B auf. Der zweite Zustandsraum Z2 weist die zweiten physiologischen Zustände Z2.A und Z2.B auf. Der erste Zustandsraum Z1 und der zweite Zustandsraum Z2 spannen zusammen den dritten Zustandsraum Z3 auf, wobei jede der mehreren abgetasteten Zustandskombinationen A1, A2 einen Zustand Z1.A, Z1.B aus dem ersten Zustandsraum Z1 und einen Zustand Z2.A, Z2.B aus dem zweiten Zustandsraum Z2 aufweist. Der dritte Zustandsraum Z3 weist die Menge MA an mehreren abgetasteten Zustandskombinationen A1, A2 auf. Die virtuelle Zustandskombination V liegt innerhalb des dritten Zustandsraums Z3 außerhalb der Menge MA an mehreren abgetasteten Zustandskombinationen A1, A2.

Für die Menge MA an mehreren abgetasteten Zustandskombinationen A1, A2 liegen die medizinischen Messdaten, beispielsweise Rohdaten oder die mehreren Bilddatenvorlagen, vor. Das medizinische Bild weist die virtuelle Zustandskombination V auf.

In Fig. 3 wird das Ergebnis des Ausführungsbeispiels angezeigt, wenn die medizinischen Messdaten solange erfasst werden, bis die medizinischen Messdaten jeden Zustand Z1.A, Z1.B aus dem ersten Zustandsraum Z1 und jeden Zustand Z2.A, Z2.B aus dem zweiten Zustandsraum Z2 einmal aufweisen.

Diese schematische Abbildung entspricht auch demjenigen Ausführungsbeispiel, wenn die medizinischen Messdaten solange erfasst werden, bis die medizinischen Messdaten jeden Zustand Z1.A, Z1.B aus dem ersten Zustandsraum Z1 und jeden Zustand Z2.A, Z2.B aus dem zweiten Zustandsraum Z2 lediglich einmal aufweisen.

Die Menge MA an mehreren abgetasteten Zustandskombinationen A1, A2 füllt den dritten Zustandsraum Z3 maximal bis einschließlich 50 Prozent auf.

**Fig. 4** zeigt eine schematische Abbildung des dritten Zustandsraums Z3 in einem dritten Ausführungsbeispiel. Mehrere medizinische Bilder des Patienten P werden unter Verwendung der medizinischen Messdaten erzeugt, wobei die mehreren medizinischen Bilder eine Menge MV an mehreren virtuellen Zustandskombinationen V1, V2 aufweisen, wobei jede der mehreren virtuellen Zustandskombinationen V1, V2 einen Zustand Z1.A, Z1.B aus dem ersten Zustandsraum Z1 und einen Zustand Z2.A, Z2.B aus dem zweiten Zustandsraum Z2 aufweist, wobei der dritte Zustandsraum Z3 die Menge MV an mehreren virtuellen Zustandskombinationen V1, V2 aufweist und wobei die Menge MV an mehreren virtuellen Zustandskombinationen V1, V2 innerhalb des dritten Zustandsraum Z3 außerhalb der Menge MA an mehreren abgetasteten Zustandskombinationen A1, A2 liegt.

Grundsätzlich ist es denkbar, dass die mehreren medizinischen Bilder der Menge MV der mehreren virtuellen Zustandskombinationen gemeinsam mit den medizinischen Messdaten, insbesondere den mehreren Bilddatenvorlagen, der Menge MA der mehreren abgetasteten Zustandskombinationen bereitgestellt werden.

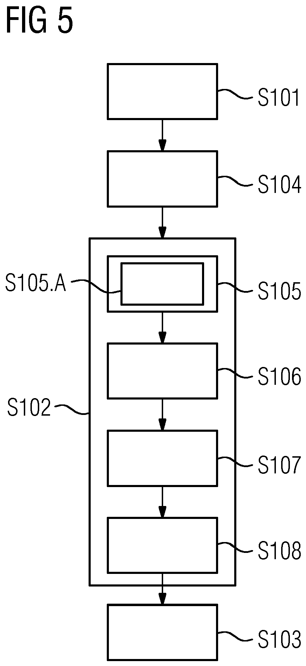

**Fig. 5** zeigt ein erstes Flussdiagramm eines Verfahrens für ein Bereitstellen eines medizinischen Bildes eines Patienten in einem vierten Ausführungsbeispiel. Die in Fig. 5 gezeigten Verfahrensschritte S104 bis S108 können quasi beliebig kombiniert werden.

Verfahrensschritt S104 kennzeichnet, dass unter Verwendung der medizinischen Messdaten mehrere Bilddatenvorlagen rekonstruiert werden, wobei das medizinische Bild unter Verwendung der mehreren Bilddatenvorlagen erzeugt wird.

Verfahrensschritt S105 kennzeichnet das Ermitteln eines Deformationsfelds unter Verwendung der medizinischen Messdaten, insbesondere der mehreren Bilddatenvorlagen, wobei die virtuelle Zustandskombination beim Ermitteln als Eingangsparameter eingesetzt wird, wobei das Deformationsfeld jeweils paarweise für die Menge der mehreren abgetasteten Zustandskombinationen und/oder für eine interpolierte Zustandskombination aus der Menge der mehreren abgetasteten Zustandskombinationen ermittelt wird.

Verfahrensschritt S105.A kennzeichnet, dass das Ermitteln des Deformationsfeldes ein Modellieren und/oder ein Registrieren der medizinischen Messdaten, insbesondere der mehreren Bilddatenvorlagen, umfasst.

Verfahrensschritt S106 kennzeichnet das Anwenden des Deformationsfelds auf die medizinischen Messdaten, insbesondere auf die mehreren Bilddatenvorlagen.

Verfahrensschritt S107 kennzeichnet das Aufteilen der medizinischen Messdaten, insbesondere der mehreren Bilddatenvorlagen, in einen niederfrequenten Messdatenanteil und in einen hochfrequenten Messdatenanteil nach dem Anwenden des Deformationsfelds.

Verfahrensschritt S108 kennzeichnet das Kombinieren des niederfrequenten Messdatenanteils und des hochfrequenten Messdatenanteils mit unterschiedlichen Gewichtungen, wodurch das medizinische Bild erzeugt wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Bereitstellen eines medizinischen Bildes eines Patienten, mit den folgenden Schritten:
- Erfassen von medizinischen Messdaten des Patienten mittels eines Computertomographen,
-- wobei die medizinischen Messdaten eine Menge an mehreren abgetasteten Zustandskombinationen aufweisen,
-- wobei ein erster Zustandsraum erste physiologische Zustände des Patienten aufweist, wobei ein zweiter Zustandsraum zweite physiologische Zustände des Patienten aufweist und wobei der erste Zustandsraum und der zweite Zustandsraum zusammen einen dritten Zustandsraum aufspannen,
-- wobei jede der mehreren abgetasteten Zustandskombinationen einen Zustand aus dem ersten Zustandsraum und einen Zustand aus dem zweiten Zustandsraum aufweist,
-- wobei der dritte Zustandsraum die Menge an mehreren abgetasteten Zustandskombinationen aufweist,
-- wobei die ersten physiologischen Zustände und die zweiten physiologischen Zustände aus der folgenden Liste ausgewählt werden:
--- mehrere Atemzustände einer Atmung des Patienten,
--- mehrere Kontrastmittelzustände einer Kontrastmittelanreicherung im Patienten,
--- mehrere kardiovaskuläre Zustände eines Herzens des Patienten,
--- mehrere morphologische Zustände eines Tumors des Patienten,
--- mehrere funktionelle Zustände eines Organs des Patienten,
--- mehrere Gelenkzustände einer Extremität des Patienten,
- Festlegen einer virtuellen Zustandskombination durch einen Nutzer,
-- wobei die virtuelle Zustandskombination nicht Teil der medizinischen Messdaten ist,
-- wobei die virtuelle Zustandskombination einen Zustand aus dem ersten Zustandsraum und einen Zustand aus dem zweiten Zustandsraum aufweist,
-- wobei der dritte Zustandsraum die virtuelle Zustandskombination aufweist und
-- wobei die virtuelle Zustandskombination innerhalb des dritten Zustandsraums außerhalb der Menge an mehreren abgetasteten Zustandskombinationen liegt,
- Erzeugen eines medizinischen Bildes des Patienten,
-- wobei ein Deformationsfeld unter Verwendung der medizinischen Messdaten ermittelt wird, wobei die durch den Nutzer festgelegte virtuelle Zustandskombination beim Ermitteln des Deformationsfelds als Eingangsparameter eingesetzt wird, wobei ein Registrieren der medizinischen Messdaten das Ermitteln des Deformationsfeldes ermöglicht, wobei das Deformationsfeld jeweils paarweise für die Menge der mehreren abgetasteten Zustandskombinationen und/oder für eine interpolierte Zustandskombination aus der Menge der mehreren abgetasteten Zustandskombinationen ermittelt wird, wobei das Deformationsfeld einer Interpolation oder einer Extrapolation der medizinischen Messdaten entspricht und wobei die medizinischen Messdaten derart durch ein Anwenden des Deformationsfelds auf die medizinischen Messdaten verformt werden, dass das erzeugte medizinische Bild die virtuelle Zustandskombination aufweist, und
- Bereitstellen des erzeugten medizinischen Bildes des Patienten.

2. Verfahren nach Anspruch 1, wobei das medizinische Bild des Patienten derart unter Verwendung der medizinischen Messdaten erzeugt wird, dass die virtuelle Zustandskombination und eine Zustandskombination aus der Menge der mehreren abgetasteten Zustandskombinationen denselben Zustand aus dem ersten Zustandsraum und/oder denselben Zustand aus dem zweiten Zustandsraum aufweisen.

3. Verfahren nach einem der vorhergehenden Ansprüche, wobei die medizinischen Messdaten solange erfasst werden, bis die medizinischen Messdaten jeden Zustand aus dem ersten Zustandsraum und/oder jeden Zustand aus dem zweiten Zustandsraum einmal aufweisen.

4. Verfahren nach einem der vorhergehenden Ansprüche, wobei die medizinischen Messdaten solange erfasst werden, bis die medizinischen Messdaten jeden Zustand aus dem ersten Zustandsraum und jeden Zustand aus dem zweiten Zustandsraum lediglich einmal aufweisen.

5. Verfahren nach einem der vorhergehenden Ansprüche, wobei der erste Zustandsraum die mehreren Atemzustände der Atmung des Patienten aufweist und wobei der zweite Zustandsraum die mehreren Kontrastmittelzustände der Kontrastmittelanreicherung im Patienten aufweist.

6. Verfahren nach einem der vorhergehenden Ansprüche, wobei die Menge an mehreren abgetasteten Zustandskombinationen den dritten Zustandsraum maximal bis einschließlich 50 Prozent auffüllt.

7. Verfahren nach einem der vorhergehenden Ansprüche, wobei unter Verwendung der medizinischen Messdaten mehrere Bilddatenvorlagen rekonstruiert werden und wobei das medizinische Bild unter Verwendung der mehreren Bilddatenvorlagen erzeugt wird.

8. Verfahren nach einem der vorhergehenden Ansprüche, wobei mehrere medizinische Bilder des Patienten unter Verwendung der medizinischen Messdaten erzeugt werden, wobei die mehreren medizinischen Bilder eine Menge an mehreren virtuellen Zustandskombinationen aufweisen, wobei jede der mehreren virtuellen Zustandskombinationen einen Zustand aus dem ersten Zustandsraum und einen Zustand aus dem zweiten Zustandsraum aufweist, wobei der dritte Zustandsraum die Menge an mehreren virtuellen Zustandskombinationen aufweist und wobei die Menge an mehreren virtuellen Zustandskombinationen innerhalb des dritten Zustandsraum außerhalb der Menge an mehreren abgetasteten Zustandskombinationen liegt.

9. Verfahren nach einem der vorhergehenden Ansprüche, wobei das Erzeugen des medizinischen Bildes weitere folgende Schritte umfasst:
- Aufteilen der medizinischen Messdaten in einen niederfrequenten Messdatenanteil und in einen hochfrequenten Messdatenanteil nach dem Anwenden des Deformationsfelds und
- Kombinieren des niederfrequenten Messdatenanteils und des hochfrequenten Messdatenanteils mit unterschiedlichen Gewichtungen, wodurch das medizinische Bild erzeugt wird.

10. Anlage für ein Bereitstellen eines medizinischen Bildes eines Patienten, aufweisend
- eine Recheneinheit, wobei die Recheneinheit zu einem Ausführen eines Verfahrens nach einem der Ansprüche 1 bis 9 ausgebildet ist, wobei die Anlage einen Computertomographen aufweist, welcher zum Erfassen der medizinischen Messdaten des Patienten ausgebildet ist.

11. Computerprogrammprodukt, aufweisend Programmcodemittel, welche in eine Recheneinheit ladbar sind, um ein Verfahren nach einem der Ansprüche 1 bis 9 auszuführen, wenn die Programmcodemittel in der Recheneinheit ausgeführt werden.

## Claims

1. Method for providing a medical image of a patient, comprising the following steps:
- acquiring medical measurement data of the patient using a computed tomography system,
-- wherein the medical measurement data has a set of multiple sampled state combinations,
-- wherein a first state space has first physiological states of the patient, wherein a second state space has second physiological states of the patient, and wherein the first state space and the second state space together span a third state space,
-- wherein each of the multiple sampled state combinations has a state from the first state space and a state from the second state space,
-- wherein the third state space has the set of multiple sampled state combinations,
-- wherein the first physiological states and the second physiological states are selected from the following list:
--- multiple respiratory states of the patient's breathing,
--- multiple contrast agent states of a contrast agent accumulation in the patient,
--- multiple cardiovascular states of the patient's heart,
--- multiple morphological states of a tumour of the patient,
--- multiple functional states of an organ of the patient,
--- multiple joint states of an extremity of the patient,
- defining a virtual state combination by means of a user,
-- wherein the virtual state combination is not part of the medical measurement data,
-- wherein the virtual state combination has a state from the first state space and a state from the second state space,
-- wherein the third state space has the virtual state combination and
-- wherein the virtual state combination lies within the third state space outside of the set of multiple sampled state combinations,
- generating a medical image of the patient,
-- wherein a deformation field is determined using the medical measurement data, wherein the virtual state combination defined by the user is used as input parameters when the deformation field is determined, wherein a registration of the medical measurement data enables the determination of the deformation field, wherein the deformation field is in each case determined in pairs for the set of multiple sampled state combinations and/or for an interpolated state combination from the set of multiple sampled state combinations, wherein the deformation field corresponds to an interpolation or an extrapolation of the medical measurement data and wherein the medical measurement data is deformed by applying the deformation field to the medical measurement data in such a way that the medical image generated has the virtual state combination, and
- providing the generated medical image of the patient.

2. Method according to claim 1, wherein the medical image of the patient is generated using the medical measurement data in such a way that the virtual state combination and a state combination from the set of multiple sampled state combinations contain the same state from the first state space and/or the same state from the second state space.

3. Method according to one of the preceding claims, wherein the medical measurement data continues to be acquired until the medical measurement data contains each state from the first state space and/or each state from the second state space once.

4. Method according to one of the preceding claims, wherein the medical measurement data continues to be acquired until the medical measurement data contains each state from the first state space and each state from the second state space only once.

5. Method according to one of the preceding claims, wherein the first state space comprises the multiple respiratory states of the patient's breathing and wherein the second state space comprises the multiple contrast agent states of the contrast agent accumulation in the patient.

6. Method according to one of the preceding claims, wherein the set of multiple sampled state combinations fills the third state space by up to and including 50 percent at a maximum.

7. Method according to one of the preceding claims, wherein multiple image data templates are reconstructed using the medical measurement data and wherein the medical image is generated using the multiple image data templates.

8. Method according to one of the preceding claims, wherein multiple medical images of the patient are generated using the medical measurement data, wherein the multiple medical images have a set of multiple virtual state combinations, wherein each of the multiple virtual state combinations contains a state from the first state space and a state from the second state space, wherein the third state space has the set of multiple virtual state combinations, and wherein the set of multiple virtual state combinations lies within the third state space outside the set of multiple sampled state combinations.

9. Method according to one of the preceding claims, wherein the generation of the medical image comprises the following further steps:
- subdividing the medical measurement data into a low-frequency measurement data portion and a high-frequency measurement data portion following the application of the deformation field, and
- combining the low-frequency measurement data portion and the high-frequency measurement data portion with different weightings, thereby generating the medical image.

10. System for providing a medical image of a patient, having - a computing unit, wherein the computing unit is embodied for performing a method according to one of claims 1 to 9, wherein the system has a computed tomography system which is embodied for acquiring the medical measurement data of the patient.

11. Computer program product having program code means which can be loaded into a computing unit in order to perform a method according to one of claims 1 to 9 when the program code means are executed in the computing unit.

## Revendications

1. Procédé pour se procurer une image médicale d'un patient, comprenant les stades suivants :
- détection de données de mesure médicales du patient au moyen d'un tomographe à ordinateur,
-- dans lequel les données de mesure médicales ont un ensemble de plusieurs combinaisons d'état échantillonnées,
-- dans lequel un premier espace d'état a des premiers états physiologiques du patient, un deuxième espace d'état ayant des deuxièmes états physiologiques du patient et le premier espace d'état et le deuxième espace d'état engendrant ensemble un troisième espace d'état,
-- chacune des plusieurs combinaisons d'état échantillonnées a un état provenant du premier espace d'état et un état composé du deuxième espace d'état,
-- le troisième espace d'état ayant l'ensemble de plusieurs combinaisons d'état échantillonnées,
-- les premiers états physiologiques et les deuxièmes états physiologiques sont choisis dans la liste suivante :
--- plusieurs états respiratoires d'une respiration du patient,
--- plusieurs états d'agent de contraste d'un enrichissement en agent de contraste dans le patient,
--- plusieurs états cardiovasculaires du cœur du patient,
--- plusieurs états morphologiques d'une tumeur du patient,
--- plusieurs états fonctionnels d'un organe du patient,
--- plusieurs état d'articulation d'une extrémité du patient,
- fixation d'une combinaison d'état virtuelle par un utilisateur,
-- la combinaison d'état virtuelle ne faisant pas partie des données de mesure médicales,
-- la combinaison d'état virtuelle ayant un état provenant du premier espace d'état et un état provenant du deuxième espace d'état,
-- le troisième espace d'état ayant la combinaison d'état virtuelle et
-- la combinaison d'état virtuelle à l'intérieur du troisième espace d'état se trouvant à l'extérieur de l'ensemble de plusieurs combinaisons d'état échantillonnées,
- production d'une image médicale du patient,
-- un champ de déformation étant déterminé en utilisant les données de mesure médicales, la combinaison d'état virtuelle fixée par l'utilisateur étant, lors de la détermination du champ de déformation, utilisée comme paramètre d'entrée, un enregistrement des données de mesure médicales rendant possible la détermination du champ de déformation, le champ de déformation étant déterminé par paire pour l'ensemble des plusieurs combinaisons d'état échantillonnées et/ou pour une combinaison d'état interpolée à partir de l'ensemble des plusieurs combinaisons d'état échantillonnées, le champ de déformation correspondant à une interpolation ou à une extrapolation des données de mesure médicales et les données de mesure médicales étant déformées par une application du champ de déformation aux données de mesure médicales, de manière à ce que l'image médicale produite ait la combinaison d'état virtuelle, et
- mise à disposition de l'image médicale produite du patient.

2. Procédé suivant la revendication 1, dans lequel on produit l'image médicale du patient en utilisant les données de mesure médicales, de manière à ce que la combinaison d'état virtuelle et une combinaison d'état provenant de l'ensemble des plusieurs combinaisons d'état échantillonnées aient le même état provenant du premier espace d'état et/ou le même état provenant du deuxième espace d'état.

3. Procédé suivant l'une des revendications précédentes, dans lequel on détecte les données de mesure médicales jusqu'à ce que les données de mesure médicales aient une fois chaque état provenant du premier espace d'état et/ou chaque état provenant du deuxième espace d'état.

4. Procédé suivant l'une des revendications précédentes, dans lequel on détecte les données de mesure médicales jusqu'à ce que les données de mesure médicales aient, une fois seulement, chaque état provenant du premier espace d'état et chaque état provenant du deuxième espace d'état.

5. Procédé suivant l'une des revendications précédentes, dans lequel le premier espace d'état a les plusieurs états respiratoires de la respiration du patient et dans lequel le deuxième espace d'état a les plusieurs états d'agent de contraste de l'enrichissement en agent de contraste dans le patient.

6. Procédé suivant l'une des revendications précédentes, dans lequel l'ensemble des plusieurs combinaisons d'état échantillonnées remplit le troisième espace d'état au maximum jusqu'à y compris 50 pour cent.

7. Procédé suivant l'une des revendications précédentes, dans lequel, en utilisant les données de mesure médicales, on reconstruit plusieurs originaux de données d'image et dans lequel on produit l'image médicale en utilisant les plusieurs originaux de données d'image.

8. Procédé suivant l'une des revendications précédentes, dans lequel on produit plusieurs images médicales du patient en utilisant les données de mesure médicales, les plusieurs images médicales ayant un ensemble de plusieurs combinaisons d'état virtuelles, chacune des plusieurs combinaisons d'état virtuelles ayant un état provenant du premier espace d'état et un état provenant du deuxième espace d'état, le troisième espace d'état ayant l'ensemble de plusieurs combinaisons d'état virtuelles et l'ensemble de plusieurs combinaisons d'état virtuelles, à l'intérieur du troisième espace d'état, étant à l'extérieur de l'ensemble de plusieurs combinaisons d'état échantillonnées.

9. Procédé suivant l'une des revendications précédentes, dans lequel la production de l'image médicale comprend les autres stades suivants :
- subdivision des données de mesure médicales en une partie de données médicales à basse fréquence et en une partie de données médicales à haute fréquence après l'application du champ de déformation et
- combinaisons de la partie de données de mesure de basse fréquence et de la partie de données de mesure de haute fréquence à des pondérations différentes, grâce à quoi on produit l'image médicale.

10. Installation de mise à disposition d'une image médicale d'un patient, comportant
- une unité informatique, l'unité informatique étant constituée pour une exécution d'un procédé suivant l'une des revendications 1 à 9, l'installation ayant un tomographe à ordinateur constitué pour détecter les données de mesure médicales du patient.

11. Produit de programme d'ordinateur ayant des moyens de code de programme, qui peuvent être chargés dans une unité informatique, pour exécuter un procédé suivant l'une des revendications 1 à 9, lorsque les moyens de code de programme sont réalisés dans l'unité informatique.
